# EUROPEAN PATENT APPLICATION

(11) **EP 4 736 908 A1**
(43) Date of publication of application: **06.05.2026**
(21) Application number: 24856199.5
(22) Date of filing: 23.07.2024
(51) Int. Cl.: A61M 5/142

(54) **PHARMACEUTICAL LIQUID ADMINISTRATION DEVICE**

(30) Priority: 23.08.2023 JP 2023135305
(71) Applicant: Terumo Kabushiki Kaisha, Tokyo 151-0072 (JP)
(72) Inventor: SEKIGUCHI, Shota, Ashigarakami-gun, Kanagawa 259-0151 (JP); UCHIYAMA, Joji, Ashigarakami-gun, Kanagawa 259-0151 (JP); YAMAZAKI, Tsuyoshi, Ashigarakami-gun, Kanagawa 259-0151 (JP)
(74) Representative: TBK
(86) International application number: PCT/JP2024/026240
(87) International publication number: WO 2025/041505

(57) **Abstract**

A drug solution administration device (10) includes: a holder (20); a cannula unit (60) that is provided on the holder; and a device main body (100) that is detachably mounted to the holder. The device main body includes a casing (120). One of the holder and the casing includes a guide rib (44). Another of the holder and the casing includes a guide groove (136) with which the guide rib engages in a relatively movable manner. At least one of the guide rib and the guide groove includes an inclined portion (140). When the device main body is attached to or detached from the holder, the guide rib and the inclined portion guide the device main body in an inclined direction along the inclined portion.

## Description

### Technical Field

The present invention relates to a drug solution administration device for administering a drug solution to a living subject.

### Background Art

As illustrated particularly in Figs. 1 and 2 of WO 2016/132936 A, a portable drug solution administration device includes a holder having a cannula and a device main body mounted to the holder. In WO 2016/132936 A, the holder is referred to as "cradle". The device main body includes a casing that accommodates a reservoir and a plunger therein.

A user of the drug solution administration device fixes the holder to the body of a patient by adhering it thereto. Next, the user causes the distal end of the cannula to protrude from the holder and indwell in the body of the patient. Subsequently, after filling the reservoir with the drug solution, the user mounts the device main body on the holder. Thereafter, the plunger moves inside the reservoir toward the distal end. The drug solution inside the reservoir is pushed out from the reservoir by the moving plunger. The drug solution pushed out from the reservoir is administered into the body of the patient through the cannula.

When continuing the administration of the drug solution to the patient, the user replaces the device main body with a new device main body in which the reservoir is filled with the drug solution. At this time, first, the used device main body is detached from the holder. Next, the device main body is mounted to the holder again. Accordingly, before and after the replacement of the device main body, the holder remains fixed to the body of the patient, and the distal end of the cannula remains indwelled in the body of the patient.

### Summary of Invention

When the user removes the device main body from the holder, there is a concern that the casing constituting the device main body may interfere with the cannula.

An object of the present invention is to solve the above-described problem.

(1) An aspect of the present invention provides a drug solution administration device including: a holder that is adhered to a body of a living subject; a cannula unit that is provided on the holder; and a device main body that is detachably mounted to the holder, wherein the device main body includes a reservoir which is filled with a drug solution to be supplied to a cannula of the cannula unit; and a casing which accommodates the reservoir, the holder includes a base portion, the base portion includes a holder outer surface which faces the body when the holder is adhered to the body; and a holder inner surface that is on a side opposite to the holder outer surface and faces the device main body when the device main body is mounted to the holder, the cannula unit includes a housing which is attached to the holder inner surface in a state of protruding in a thickness direction of the base portion from the holder outer surface; and a first connecting portion provided on the housing, the device main body includes a second connecting portion facing the first connecting portion, the first connecting portion and the second connecting portion are connected to each other by relative movement of the device main body with respect to the holder along the holder inner surface in a connection direction, the first connecting portion and the second connecting portion are detached from each other by relative movement of the device main body with respect to the holder along the holder inner surface in a detachment direction, which is opposite to the connection direction, one of the holder and the casing includes a guide rib, another of the holder and the casing includes a guide groove with which the guide rib engages in a relatively movable manner, at least one of the guide rib and the guide groove includes an inclined portion inclined in a direction away from the base portion as the inclined portion extends in the detachment direction, and when the device main body is attached to or detached from the holder, the guide rib and the inclined portion guide the device main body in an inclined direction along the inclined portion.

In the above configuration, when the device main body is attached to or detached from the holder, the guide rib relatively moves along the guide groove. During this movement, the guide rib and the inclined portion guide the device main body in a direction inclined with respect to the detachment direction. That is, when the device main body is attached to or detached from the holder, the moving direction of the device main body is regulated. As a result, interference of the casing with the cannula unit is prevented, thereby preventing detachment of the cannula unit from the holder.

(2) In the drug solution administration device according to item (1) above, the holder may include two holder side portions which protrude from the base portion in the thickness direction and are provided at positions sandwiching the casing when the device main body is mounted to the holder, each of the two holder side portions may include the guide rib or the guide groove.

By guiding the two guide ribs with the two inclined portions, the moving direction of the device main body can be regulated more effectively.

(3) In the drug solution administration device according to item (1) or (2) above, the holder or the casing may include an engagement recess which is continuous with an end portion of the guide groove facing the detachment direction and extends in the detachment direction, and when the first connecting portion and the second connecting portion are connected to each other, the guide rib may engage with the engagement recess.

When the device main body is mounted to a main body of the holder, the guide rib engages with the engagement recess. This engagement prevents the device main body from detaching from the holder. Furthermore, by relatively moving the guide rib along the engagement recess, the first connecting portion and the second connecting portion can be easily connected and detached.

(4) In the drug solution administration device according to any one of items (1) to (3) above, one of the holder and the casing may include a protrusion, and another of the holder and the casing may include a recess into which the protrusion is inserted in a relatively movable manner in the inclined direction.

According to this configuration, when the device main body is attached to or detached from the holder, it is possible to guide the protrusion along the inner surface of the recess. In this case, the moving direction of the device main body with respect to the holder is regulated more effectively. As a result, interference of the cannula unit with the casing is further prevented.

(5) In the drug solution administration device according to any one of items (1) to (4) above, the device main body may be detached from the holder by sliding along the holder inner surface in the detachment direction so that at least a part of the device main body protrudes from an end portion of the holder in the detachment direction, and then moving in a direction inclined with respect to the detachment direction.

As a result, interference of the casing with the cannula unit is further prevented. Therefore, detachment of the cannula unit from the holder is further prevented.

(6) In the drug solution administration device according to any one of items (1) to (5) above, the holder may include a protection portion which protrudes in the thickness direction from the holder inner surface, the casing may include an insertion recess into which the protection portion is inserted in a detachable manner, in a state where the device main body is mounted to the holder, the protection portion may be positioned between an end wall portion constituting an end portion of the insertion recess in the connection direction and the housing of the cannula unit, and when a protruding direction of the protection portion from the holder inner surface is defined as a height direction, a height of a top portion of the protection portion may be equal to or greater than a height of a top portion of the housing.

When the user moves the device main body in the detachment direction, the protection portion comes into contact with the end wall portion of the insertion recess. Consequently, the device main body stops, and thus, further movement of the device main body in the detachment direction is prevented. Therefore, interference of the end wall portion, which is a part of the casing, with the cannula unit is prevented.

(7) In the drug solution administration device according to item (6) above, an end surface of the protection portion facing the connection direction may be an inclined surface inclined in the detachment direction from the holder inner surface toward the top portion of the protection portion.

After the end wall portion comes into contact with the protection portion, the end surface (inclined surface) of the protection portion facing the connection direction guides the device main body in a direction inclined with respect to the detachment direction. Accordingly, for example, even after the rib has been detached from the guide groove, the moving direction of the device main body is regulated. As a result, interference of the end wall portion with the cannula unit is prevented.

(8) In the drug solution administration device according to any one of items (1) to (7) above, the cannula unit may be positioned, in the base portion, between two end portions in an axial direction which extends in the connection direction and the detachment direction, and between two end portions in a width direction orthogonal to the axial direction.

Even when the cannula is arranged at the above-described position, interference of the casing with the cannula unit can be prevented when the device main body is attached to or detached from the holder.

According to the present invention, when the device main body is attached to or detached from the holder, the guide rib and the inclined portion guide the device main body in an inclined direction along the inclined portion. That is, when the device main body is attached to or detached from the holder, the moving direction of the device main body is regulated. As a result, interference of the casing with a needle unit is prevented, and thus, detachment of the cannula unit from the holder is prevented.

### Brief Description of Drawings

Fig. 1 is an overall schematic side view of a drug solution administration device according to an embodiment of the present invention.
Fig. 2 is an overall schematic perspective view of a holder constituting the drug solution administration device.
Fig. 3 is a schematic side sectional view of the drug solution administration device viewed from a width direction.
Fig. 4 is a schematic plan view of the drug solution administration device viewed from a device main body.
Fig. 5 is a schematic perspective view of a first half constituting a casing of the device main body.
Fig. 6A is a schematic side view illustrating a position of a guide rib when the device main body is mounted to a holder. Fig. 6B is a schematic side sectional view of the drug solution administration device in the state illustrated in Fig. 6A, viewed from the width direction.
Fig. 7A is a schematic side view illustrating the position of the guide rib when the device main body is relatively moved in a detachment direction with respect to the holder. Fig. 7B is a schematic side sectional view of the drug solution administration device in the state illustrated in Fig. 7A, viewed from the width direction.
Fig. 8A is a schematic side view illustrating the position of the guide rib when the device main body moves obliquely with respect to the holder. Fig. 8B is a schematic side sectional view of the drug solution administration device in the state illustrated in Fig. 8A, viewed from the width direction.
Fig. 9A is a schematic side view illustrating the position of the guide rib when the device main body further moves obliquely from the state illustrated in Fig. 8A with respect to the holder. Fig. 9B is a schematic side sectional view of the drug solution administration device in the state illustrated in Fig. 9A, viewed from the width direction.

### Description of Embodiments

The term "living subject" in the present invention includes both human patients and animal patients; however, in the following description, a human patient will be exemplified as the living subject. Further, the term "user" refers to a person who fixes a drug solution administration device 10 to a body BD of the living subject. A typical example of the user is a patient, but the user is not limited to a patient.

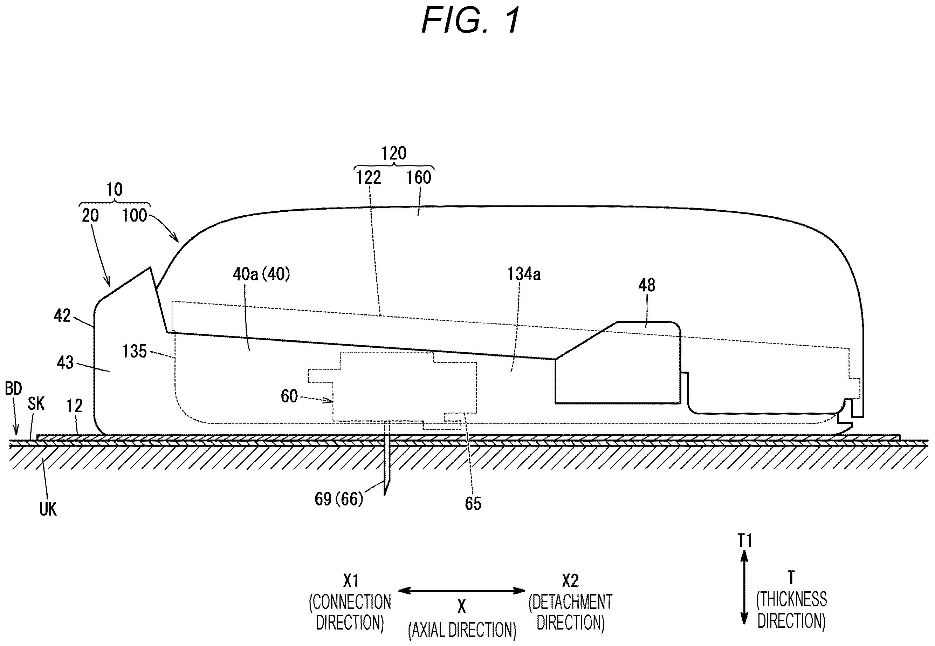

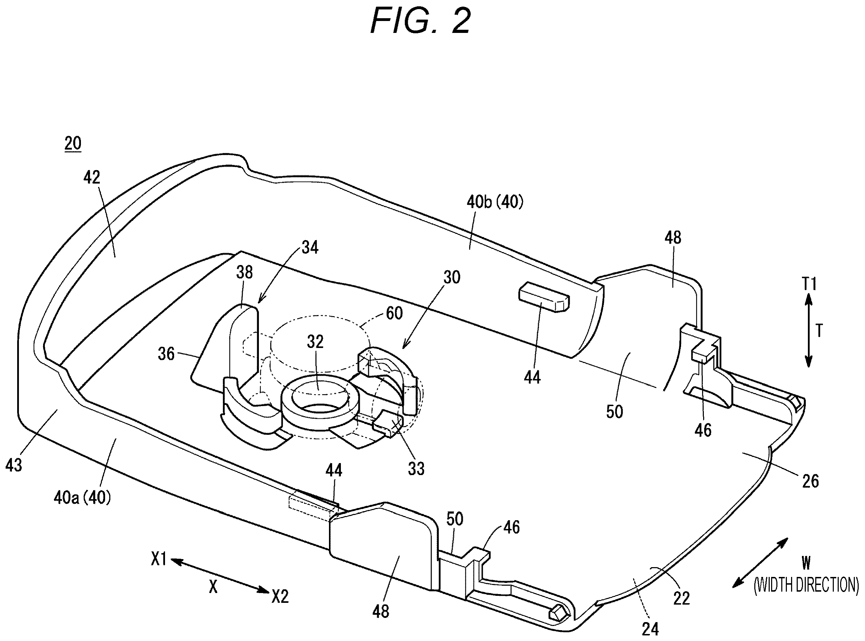

Fig. 1 is an overall schematic side view of the drug solution administration device 10. The drug solution administration device 10 is used in a state where it is fixed to the body BD of the patient. The drug solution administration device 10 is adhered, for example, to a skin SK of the patient by means of an adhesive sheet 12 provided on a holder outer surface 24 (see Fig. 2) of the drug solution administration device 10.

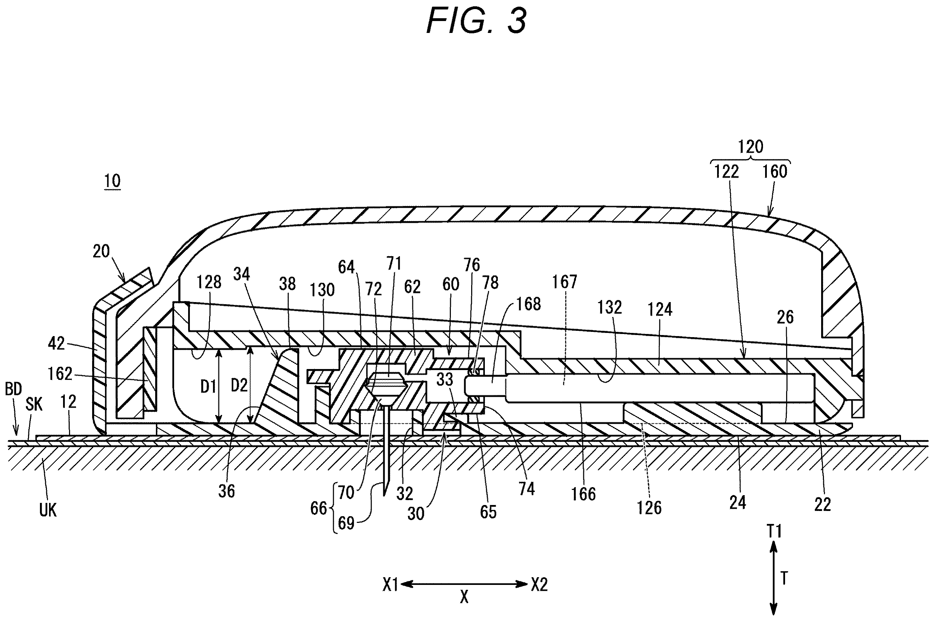

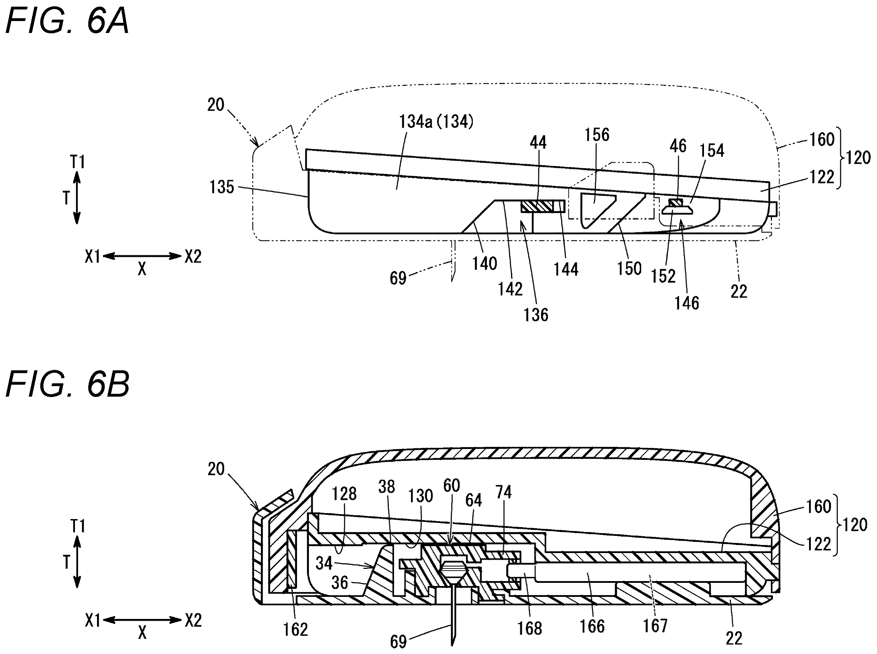

The drug solution administration device 10 includes a holder 20 and a device main body 100. As understood with reference to Figs. 6A to 9B, the device main body 100 is detachably mounted to the holder 20. When the device main body 100 is mounted to or detached from the holder 20, the device main body 100 moves relative to the holder 20 along a holder inner surface 26 (see Fig. 2). The direction of relative movement of the device main body 100 when the device main body 100 is mounted to the holder 20 is the direction in which a first connecting portion 74 (see Fig. 3) and a second connecting portion 168, which are described later, are connected to each other. This direction is hereinafter referred to as connection direction X1. The direction of relative movement of the device main body 100 when the device main body 100 is detached from the holder 20 is the direction in which the first connecting portion 74 and the second connecting portion 168 are detached from each other, and is opposite to the connection direction X1. This direction is hereinafter referred to as detachment direction X2. The direction along the connection direction X1 and the detachment direction X2 is referred to as axial direction X, and the direction orthogonal to the axial direction X in a direction parallel to the holder inner surface 26 is referred to as width direction W (see Fig. 2).

As illustrated in Fig. 2, the holder 20 includes a base portion 22. The base portion 22 includes the holder outer surface 24 and the holder inner surface 26. The adhesive sheet 12 is provided on the holder outer surface 24. When the holder 20 is adhered to the skin SK of the patient via the adhesive sheet 12, the holder outer surface 24 faces the skin SK (body BD). The holder inner surface 26 is the surface (back surface) opposite to the holder outer surface 24. Hereinafter, the direction extending from the holder outer surface 24 toward the holder inner surface 26 is referred to as thickness direction T. The thickness direction T is orthogonal to both the axial direction X and the width direction W.

The base portion 22 includes a unit holding portion 30 that protrudes from the holder inner surface 26 in the thickness direction T (height direction T1). The unit holding portion 30 holds a cannula unit 60, which is described later, on the holder 20. The unit holding portion 30 is formed with a through-hole 32 that penetrates in the thickness direction T of the base portion 22. The through-hole 32 is positioned between the two end portions of the base portion 22 in the axial direction X and between the two end portions of the base portion 22 in the width direction W. A cannula 66 (see Figs. 1 and 3) passes through the through-hole 32. The unit holding portion 30 includes a hook 33.

The base portion 22 includes a protection portion 34 that protrudes from the holder inner surface 26 in the thickness direction T. The protection portion 34 is provided on the connection direction X1 side relative to the unit holding portion 30 and extends in the thickness direction T of the base portion 22. That is, the protruding direction of the protection portion 34 is one of the directions of the thickness direction T of the base portion 22, and is a direction opposite to the skin SK. Hereinafter, this direction is referred to as height direction T1. In the protection portion 34, an end surface 36 on the connection direction X1 side is inclined toward the detachment direction X2 as the end surface 36 extends from the holder inner surface 26 toward a top portion 38 of the protection portion 34. That is, the end surface 36 is an inclined surface.

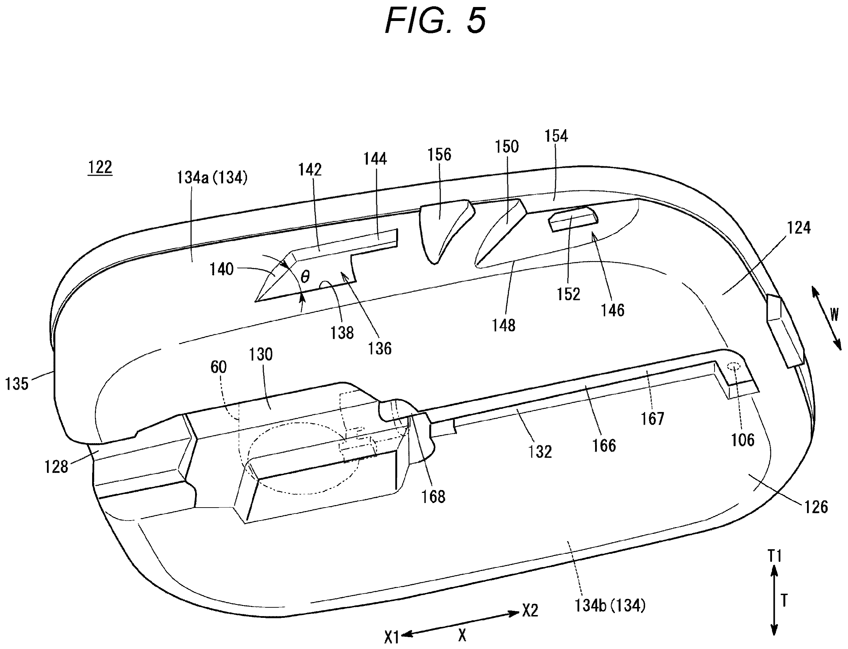

The holder 20 includes two holder side portions 40 that are continuous with the base portion 22. The two holder side portions 40 are a first side portion 40a and a second side portion 40b. The first side portion 40a and the second side portion 40b protrude in the height direction T1, similarly to the protection portion 34. When the device main body 100 is mounted to the holder 20, the first side portion 40a and the second side portion 40b sandwich a casing 120 in the width direction W. The end portion of the first side portion 40a in the connection direction X1 and the end portion of the second side portion 40b in the connection direction X1 are connected via an arch-shaped portion 42. The first side portion 40a, the second side portion 40b, and the arch-shaped portion 42 integrally form a standing wall portion 43. The standing wall portion 43 is shaped to follow a peripheral portion 135 (see Fig. 5) that includes a first side surface 134a and a second side surface 134b of the device main body 100.

In the holder 20 of the illustrated example, a guide rib 44 and a protrusion 46 are provided on the inner surface of the first side portion 40a. The guide rib 44 and the protrusion 46 protrude inward in the width direction W. The first side portion 40a includes a stepped portion 48 provided between the guide rib 44 and the protrusion 46. The stepped portion 48 of the first side portion 40a slightly protrudes outward in the width direction W relative to the outer surface of the first side portion 40a. A movement space 50 is formed inward of the stepped portion 48 of the first side portion 40a. Similarly, the second side portion 40b is also provided with the guide rib 44, the protrusion 46, and the stepped portion 48. The stepped portion 48 of the second side portion 40b also slightly protrudes outward in the width direction W relative to the outer surface of the second side portion 40b. The movement space 50 is formed inward of the stepped portion 48 of the second side portion 40b. The two guide ribs 44 are provided at positions facing each other. Similarly, the two protrusions 46 are provided at positions facing each other. In addition, the two stepped portions 48 are also provided at positions facing each other.

When administering the drug solution to the patient using the drug solution administration device 10, the cannula unit 60 is provided on the holder 20, as illustrated in Fig. 3. Specifically, the cannula unit 60 includes a housing 62. The housing 62 is attached to the unit holding portion 30 of the holder inner surface 26. The housing 62, which is attached to the unit holding portion 30 (the holder inner surface 26), is in a state of protruding from the holder inner surface 26 in the height direction T1 (the protruding direction of the protection portion 34), which is one of the directions of the thickness direction T of the base portion 22. In the illustrated example, regarding the height from the holder inner surface 26 in the height direction T1, the height of a top portion 64, which is the outer surface of the housing 62 in the height direction T1, is equal to or smaller than the height of the top portion 38 of the protection portion 34. In other words, the height of the top portion 38 of the protection portion 34 is equal to or greater than the height of the top portion 64 of the housing 62.

The housing 62 includes a locking recess 65. When the user performs a predetermined operation using a puncture device which is not illustrated and is previously mounted to the holder 20, the housing 62 rotates. In conjunction with this rotation, the hook 33 provided on the holder 20 engages with the locking recess 65. As a result, the cannula unit 60 is positioned and fixed to the unit holding portion 30.

The cannula unit 60 includes the cannula 66 provided in the housing 62. As illustrated in Fig. 3, the cannula 66 includes a main body portion 69 positioned at a distal end and a flare portion 70 positioned at a proximal end. A fixing member 71 is mounted to the upper portion of the flare portion 70. The fixing member 71 is fitted into a fitting recess 72 formed in the housing 62. By this fitting, the cannula 66 is held in the housing 62 by the fixing member 71. When the user performs a predetermined operation using a puncture tool, the fixing member 71 holding the cannula 66 approaches the unit holding portion 30, and the main body portion 69 of the cannula 66 is exposed from the through-hole 32 together with a puncture needle of the puncture device. The puncture needle and the main body portion 69 exposed from the through-hole 32 are inserted into the skin SK of the patient.

The housing 62 includes the first connecting portion 74. The first connecting portion 74 protrudes from a location of the housing 62 in the detachment direction X2. The protruding direction of the first connecting portion 74 is the detachment direction X2. A port 76 is formed inside the first connecting portion 74. The port 76 communicates with a lumen (not illustrated) of the cannula 66. A diaphragm 78 is arranged within the port 76.

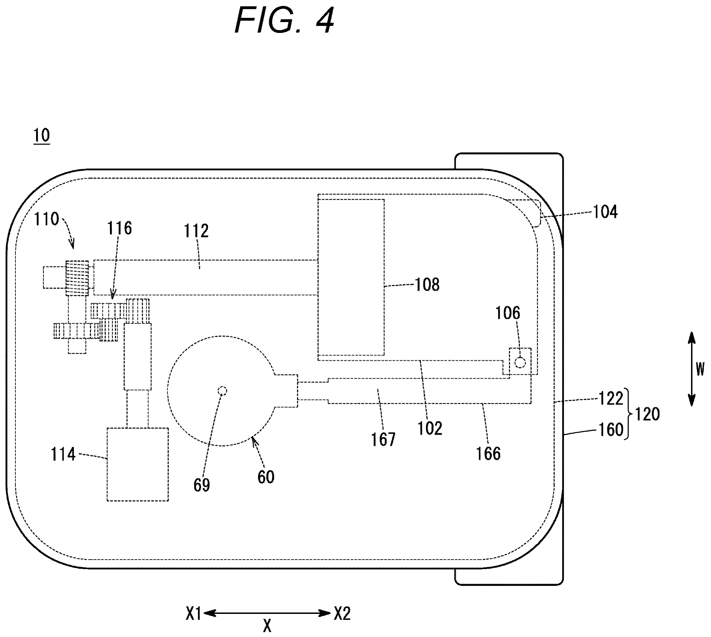

As illustrated in Fig. 4, the device main body 100 includes a reservoir 102 and the casing 120 accommodating the reservoir 102. The reservoir 102 includes a drug solution inlet 104 and a drug solution outlet 106. The drug solution is supplied into the reservoir 102 through the drug solution inlet 104 by the user performing a predetermined operation.

Inside the reservoir 102, a gasket 108 moves. A pump unit 110 is accommodated inside the casing 120. The pump unit 110 includes a plunger 112 that pushes the gasket 108, and a motor 114 that drives the plunger 112. The motor 114 and the plunger 112 are connected via a plurality of gears 116. When the motor 114 is driven, the plunger 112 advances toward the drug solution outlet 106. Along with this advancement, the drug solution inside the reservoir 102 is pushed by the gasket 108 and discharged from the drug solution outlet 106.

As illustrated in Fig. 3, the casing 120 includes a first half 122 and a second half 160. The first half 122 includes a bottom portion 124. The bottom portion 124 includes a base side portion 134 and an outer surface 126 which are described later. The base side portion 134 is erected in the height direction T1 from the peripheral portion of the outer surface 126, thereby allowing components constituting the pump unit 110 to be accommodated inside the first half 122.

The outer surface 126 of the bottom portion 124 faces the holder outer surface 24 of the base portion 22 of the holder 20 when the device main body 100 is mounted on the holder 20. As illustrated in Figs. 3 and 5, a semicircular insertion recess 128, an accommodation recess 130, and an accommodation groove 132 are formed on the outer surface 126 of the bottom portion 124. The insertion recess 128, the accommodation recess 130, and the accommodation groove 132 are arranged in this order from the connection direction X1 toward the detachment direction X2 and recessed in a direction away from the base portion 22 of the holder 20. The insertion recess 128, the accommodation recess 130, and the accommodation groove 132 form a continuous space.

As illustrated in Fig. 3, the opening at the end portion of the insertion recess 128 in the connection direction X1 is covered with an end wall portion 162 of the second half 160. As a result, the insertion recess 128 opens only at a portion facing the holder inner surface 26. As understood from the foregoing, the end wall portion 162 is the end portion of the insertion recess 128 in the connection direction X1. In a state where the device main body 100 is mounted on the holder 20, the end wall portion 162 faces the arch-shaped portion 42 of the holder 20. In the state where the device main body 100 is mounted on the holder 20, the protection portion 34 is positioned between the end wall portion 162 and the housing 62 of the cannula unit 60, and is positioned within the accommodation recess 130. When the device main body 100 is removed from the holder 20, the protection portion 34 first moves relatively from the accommodation recess 130 to the insertion recess 128.

In the state where the device main body 100 is mounted on the holder 20, the cannula unit 60 is accommodated in the accommodation recess 130. A distance (depth D2) from the outer surface 126 of the bottom portion 124 to the ceiling surface of the accommodation recess 130 is greater than a distance (depth D1) from the outer surface 126 of the bottom portion 124 to the ceiling surface of the insertion recess 128.

The device main body 100 includes a flow path forming member 166, as illustrated in Figs. 3 and 5. A groove is formed in the flow path forming member 166 in the longitudinal direction of the flow path forming member 166. Accordingly, the cross section of the flow path forming member 166 in a direction orthogonal to the longitudinal direction is substantially U-shaped. The flow path forming member 166 is accommodated in the accommodation groove 132 formed in the first half 122. The ceiling surface of the accommodation groove 132 closes the opening of the groove formed in the flow path forming member 166. As a result, a drug solution flow path 167 is formed.

An end portion of the flow path forming member 166 facing the connection direction X1 includes the second connecting portion 168 that faces the first connecting portion 74 of the cannula unit 60. As illustrated in Fig. 3, the first connecting portion 74 and the second connecting portion 168 are connected to each other. As a result, the device main body 100 is mounted to the holder 20. On the other hand, an end portion of the flow path forming member 166 facing the detachment direction X2 is connected to the drug solution outlet 106 of the reservoir 102. Thus, the drug solution outlet 106 of the reservoir 102 and the port 76 of the housing 62 are connected via the drug solution flow path 167.

As illustrated in Fig. 5, the first half 122 includes the base side portion 134. The base side portion 134 includes the first side surface 134a and the second side surface 134b. The first side surface 134a faces the inner surface of the first side portion 40a of the holder 20. The second side surface 134b faces the inner surface of the second side portion 40b of the holder 20.

A guide groove 136 is formed in the first side surface 134a. The guide groove 136 is positioned substantially at the center in the axial direction X on the first side surface 134a. The guide groove 136 is continuous with a first insertion and removal opening 138. The first insertion and removal opening 138 is formed at the boundary between the bottom portion 124 and the first side surface 134a of the first half 122 and extends in the axial direction X. When the first half 122 is viewed from the outer surface 126 side, the first insertion and removal opening 138 is formed as a notch recessed inward in the width direction W on the first side surface 134a. The first insertion and removal opening 138 opens a portion of the first side surface 134a toward the outer surface 126.

The guide groove 136 includes a first inclined portion 140 (inclined portion) that is continuous with the first insertion and removal opening 138. The first inclined portion 140 is recessed inward in the width direction W from the outer surface of the first side surface 134a, and is positioned on the connection direction X1 side among the side surfaces of the guide groove 136 surrounding the recess. When the guide groove 136 is viewed from the first side surface 134a, the first inclined portion 140 inclines in a direction away from the base portion 22 (that is, the outer surface 126 of the bottom portion 124) with respect to the axial direction X as the first inclined portion 140 extends from the connection direction X1 toward the detachment direction X2. An intersection angle θ between the first inclined portion 140 and the axial direction X is, for example, 40° to 50°, and typically 45°. Furthermore, the guide groove 136 includes a horizontal groove portion 142 that extends in the axial direction X and connects, on the height direction T1 side of the first insertion and removal opening 138, from the first inclined portion 140 toward the detachment direction X2. The horizontal groove portion 142 is a surface recessed inward in the width direction W from the outer surface of the first side surface 134a, and is a portion positioned on the height direction T1 side among the side surfaces surrounding the guide groove 136.

At the end portion of the horizontal groove portion 142 in the detachment direction X2, an engagement recess 144 is continuous therewith. The engagement recess 144 is a recess having a rectangular shape that is narrower in width than the guide groove 136, and extends in the axial direction X. The guide groove 136 and the engagement recess 144 are continuous with each other, thereby forming one recess. As will be described later, the guide rib 44 is inserted into the guide groove 136 and the engagement recess 144 in a movable manner in the X direction (see Figs. 6A, 7A, 8A, and 9A).

On the first side surface 134a, at a position on a side in the detachment direction X2 relative to the guide groove 136, a recess 146 is formed. The recess 146 is provided for insertion (clearance) of the protrusion 46. The recess 146 is formed by recessing the side surface of the first half 122. This prevents interference of the protrusion 46 with the first side surface 134a. A second insertion and removal opening 148 is continuous with the recess 146. The second insertion and removal opening 148 is formed at the boundary between the outer surface 126 and the first side surface 134a of the first half 122 and extends in the axial direction X. When the first half 122 is viewed from the outer surface 126 side, the second insertion and removal opening 148 is formed as a notch recessed inward in the width direction W on the first side surface 134a. The second insertion and removal opening 148 opens a portion of the first side surface 134a toward the outer surface 126.

The recess 146 includes a second inclined portion 150 that is substantially parallel to the first inclined portion 140 of the guide groove 136. The protrusion 46 is inserted into the recess 146 in a movable manner. An engagement portion 152 is provided inside the recess 146. The engagement portion 152 bulges outward in the width direction W of the first half 122. As illustrated in Figs. 6A, 7A, 8A, and 9A, a lock groove 154 is formed between the upper surface of the engagement portion 152 and the ceiling surface of the recess 146.

Between the guide groove 136 and the recess 146 on the first side surface 134a, a bulging portion 156 is provided. The bulging portion 156 bulges outward in the width direction W of the first half 122. The bulging portion 156 has a substantially right triangular shape.

Similarly to the first side surface 134a, the second side surface 134b is also provided with the guide groove 136, the recess 146, and the bulging portion 156.

Instead of forming the guide groove 136 on each of the first side surface 134a and the second side surface 134b, the guide rib 44 may be provided on each of the first side surface 134a and the second side surface 134b. In this case, the guide groove 136 is formed on each of the inner surface of the first side portion 40a and the inner surface of the second side portion 40b of the holder 20.

In the case where the guide rib 44 is provided on the holder 20, the position of the guide rib 44 is not limited to the first side portion 40a and the second side portion 40b. For example, the guide rib 44 may be provided on the holder inner surface 26 in the vicinity of the end portion facing the detachment direction X2. In this case, the guide groove 136 is formed to be recessed toward the second half 160 in the vicinity of the end portion facing the detachment direction X2 on the outer surface 126 of the bottom portion 124 of the first half 122, for example. In this configuration, for example, it is possible to provide the first inclined portion 140, whose height increases toward the detachment direction X2, on the guide rib 44 while keeping the depth of the guide groove 136 constant. Alternatively, the guide rib 44 may be formed into a rectangular column shape without the first inclined portion 140, while the guide groove 136 may be provided with the first inclined portion 140 whose height increases toward the detachment direction X2. Furthermore, the first inclined portion 140 may be provided on both the guide rib 44 and the guide groove 136.

On the second side surface 134b, constituent elements having the same configuration as described above are provided at positions mirror-symmetrical to the first side surface 134a with respect to the axial direction X. That is, on the second side surface 134b, the first inclined portion 140, the guide groove 136, the first insertion and removal opening 138, the horizontal groove portion 142, the bulging portion 156, the second insertion and removal opening 148, the engagement portion 152, the recess 146, and the second inclined portion 150 are provided. Further, on the holder 20, the guide rib 44, the protrusion 46, and the like are provided at positions corresponding to each of these constituent elements of the second side surface 134b. Thus, by arranging constituent elements that serve the same functions at positions symmetrical to each other with respect to the axial direction X on the first side surface 134a and the second side surface 134b, the attachment and detachment operations of the device main body 100 to and from the holder 20 can be performed stably.

The second half 160 is connected to the first half 122 to cover the entire upper part of the first half 122. As a result, the reservoir 102 and the pump unit 110 are accommodated in a hollow interior formed by the second half 160 and the first half 122. As described above, the end wall portion 162 of the second half 160 constitutes the end portion of the insertion recess 128 in the connection direction X1.

After the reservoir 102 inside the casing 120 has been filled with drug solution by the user, the drug solution administration device 10 is used as follows.

As illustrated in Fig. 3, the user adheres the holder 20 to the skin SK of a predetermined site of the patient via the adhesive sheet 12. An abdomen is exemplified as the predetermined site. At this time point, the cannula unit 60 is not yet held in the unit holding portion 30 of the holder 20. Next, the user performs a predetermined operation using a puncture device which is not illustrated. As a result, the puncture needle of the puncture device and the main body portion 69 of the cannula 66 pass through the through-hole 32 and are inserted into the skin SK of the patient. By the user rotating the housing 62 together with the puncture device, the cannula unit 60 approaches the unit holding portion 30, and the hook 33 of the holder 20 engages with the locking recess 65 of the housing 62.

Accordingly, the cannula unit 60 is positioned and fixed (locked) to the holder 20. Based on this, the state in which the main body portion 69 of the cannula 66 is inserted into a subcutaneous tissue UK of the patient is maintained. That is, the main body portion 69 of the cannula 66 is indwelled in the subcutaneous tissue UK of the patient. Thereafter, the user removes the puncture device from the holder 20. As a result of this operation, only the puncture needle is removed from the subcutaneous tissue UK. Accordingly, only the cannula 66 remains indwelled in the subcutaneous tissue UK.

Next, the user mounts the device main body 100 to the holder 20. The mounting procedure is the reverse of the detachment procedure of the device main body 100 from the holder 20. Since the detachment will be described later, the mounting procedure will be outlined only briefly.

### [First step]

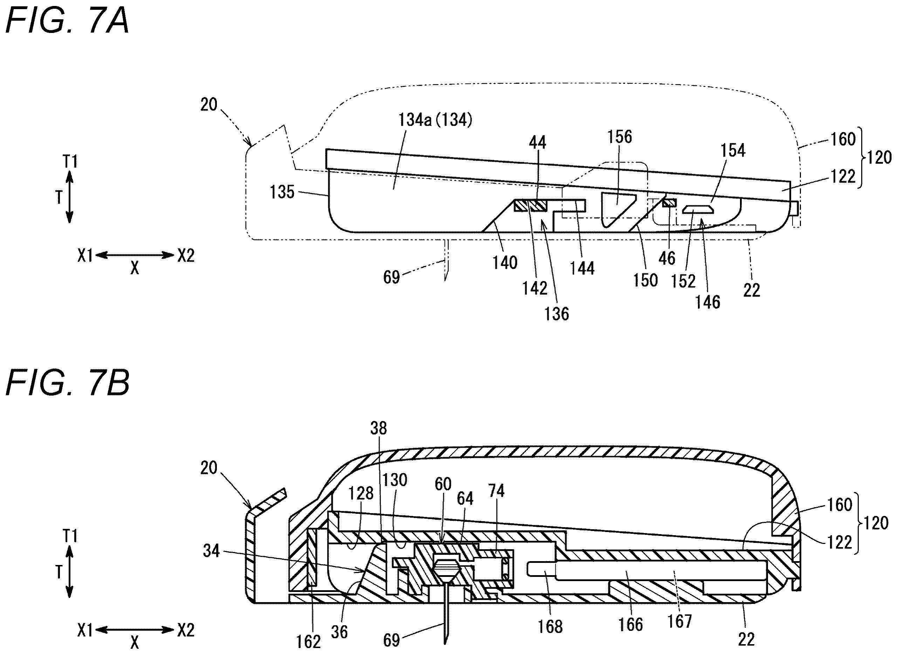

In the process of mounting the device main body 100 to the holder 20, as illustrated in Fig. 7A, the user brings the outer surface 126 of the bottom portion 124 of the first half 122 into contact with the holder inner surface 26. The user arranges the device main body 100 toward the base portion 22 (in the height direction T1) until the protection portion 34 and the cannula unit 60 are accommodated within the accommodation recess 130. At this time, in a side view from the width direction W, a portion of an end side in the detachment direction X2 of the device main body 100 protrudes in the detachment direction X2 from the upper surface of the holder 20, while another portion of the device main body 100 is placed on the holder 20.

At this time, the guide rib 44 is positioned at the end portion in the connection direction X1 of the first inclined portion 140 (or the horizontal groove portion 142) of the guide groove 136. At this time point, the first connecting portion 74 of the housing 62 and the second connecting portion 168 of the device main body 100 are not yet connected. Accordingly, the device main body 100 is not yet mounted to the holder 20. In addition, the protrusion 46 is positioned at the end portion in the connection direction X1 of the second inclined portion 150 of the recess 146.

### [Second step]

From this state, the user slides the device main body 100 on the holder 20 in the connection direction X1. The device main body 100 moves relative to the holder 20 along the holder inner surface 26 in the connection direction X1. As a result, the first connecting portion 74 of the housing 62 and the second connecting portion 168 of the flow path forming member 166 are connected to each other (see Figs. 3 and 6B). Consequently, the drug solution outlet 106 of the reservoir 102 and the port 76 of the housing 62 are connected via the drug solution flow path 167 (flow path forming member 166), and the device main body 100 is mounted to the holder 20.

Along with the above relative movement, the guide rib 44 moves relatively in the detachment direction X2 along the horizontal groove portion 142 of the guide groove 136. As illustrated in Fig. 6A, the guide rib 44 engages with the engagement recess 144 which is continuous with the horizontal groove portion 142 in the detachment direction X2. Similarly, the protrusion 46 moves relative to the recess 146 in the detachment direction X2 and enters between the upper surface of the engagement portion 152 and the ceiling surface of the recess 146. In other words, the protrusion 46 engages with the lock groove 154. Based on the engagement of the guide rib 44 with the engagement recess 144 and the engagement of the protrusion 46 with the lock groove 154, detachment of the device main body 100 from the holder 20 is prevented.

The protection portion 34 is positioned in the accommodation recess 130 in the state illustrated in Fig. 6A (see Fig. 6B), and positioned in the insertion recess 128 in the state illustrated in Fig. 7A (see Fig. 7B). The bulging portion 156 moves relatively within the movement space 50 (see Fig. 2), and comes into contact with one end of the movement space 50 in the connection direction X1 at a position where the mounting of the device main body 100 to the holder 20 is completed.

Next, the user operates, for example, a remote controller. As a result, the motor 114 (see Fig. 4) is driven, and the plunger 112 provided inside the reservoir 102 moves toward the distal end. As the plunger 112 moves, the drug solution inside the reservoir 102 is pushed by the gasket 108. Accordingly, the drug solution flows out from the drug solution outlet 106. The drug solution moves along the drug solution flow path 167, flows through the port 76 of the housing 62, and through the lumen of the cannula 66. The drug solution further flows out from the drug solution administration opening of the lumen. As a result, the drug solution is continuously administered to the patient.

When administration of the drug solution has been completed, the user removes the device main body 100 from the holder 20. First, the user moves the device main body 100 in the detachment direction X2 from the state illustrated in Fig. 6A. The device main body 100 moves relative to the holder 20 along the holder inner surface 26 in the detachment direction X2, and reaches the state illustrated in Fig. 7A. Accordingly, as illustrated in Fig. 7B, the first connecting portion 74 of the housing 62 and the second connecting portion 168 of the flow path forming member 166 are detached from each other. As a result, the device main body 100 is attached to or detached from the holder 20. As a result, in the side view from the width direction W, the portion of the end side in the detachment direction X2 of the device main body 100 protrudes in the detachment direction X2 from the upper surface of the holder 20, and another portion of the device main body 100 is placed on the holder 20.

Along with the partial sliding movement described above, the guide rib 44 is detached from the engagement recess 144, then moves relatively in the connection direction X1 along the horizontal groove portion 142 of the guide groove 136, and reaches the end portion in the connection direction X1 of the horizontal groove portion 142 (or the first inclined portion 140), as illustrated in Fig. 7A. Similarly, the protrusion 46 is detached from the lock groove 154, then moves relatively in the connection direction X1 within the recess 146, and reaches the end portion in the connection direction X1 of the second inclined portion 150. Through the above operations, the device main body 100 is released from the restraint of the holder 20. The protection portion 34 moves relatively from the accommodation recess 130 to the insertion recess 128.

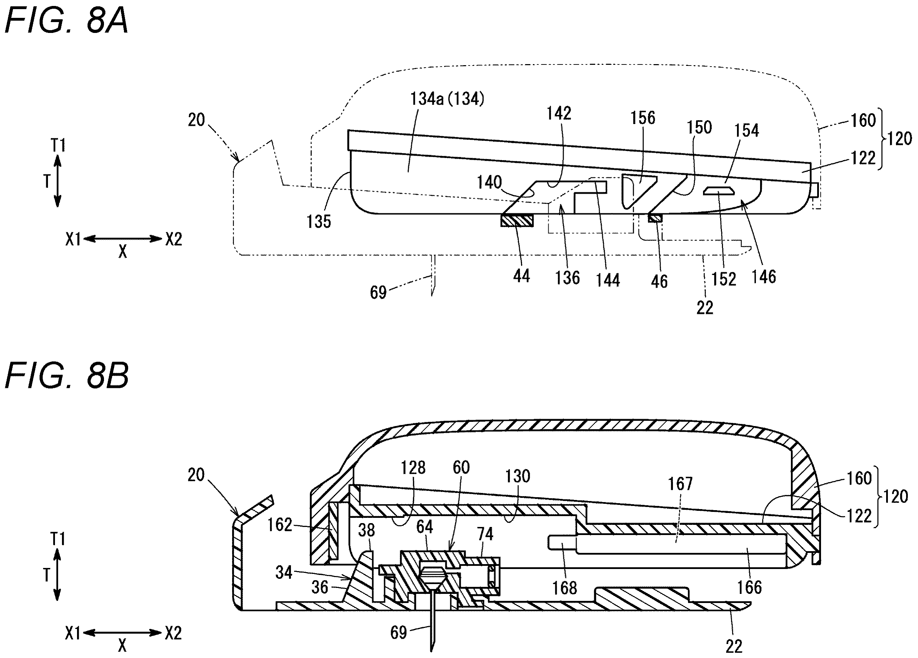

Next, the user moves the device main body 100 in a direction away from the body BD of the patient. At this time, the guide rib 44 moves relatively while sliding in contact with the inner surface of the first inclined portion 140. Accordingly, as illustrated in Fig. 8A, the device main body 100 is guided by the guide rib 44 and the first inclined portion 140 in an inclined direction along the first inclined portion 140. That is, the device main body 100 moves obliquely away from the holder 20, in a direction slanting from the height direction T1 toward the detachment direction X2. In this mode, when the device main body 100 moves obliquely, the protrusion 46 moves relatively while sliding in contact with the inner surface of the second inclined portion 150. Here, the protrusion 46 may also be separated from the inner surface of the second inclined portion 150. In such a case, the protrusion 46 does not slide in contact with the inner surface of the second inclined portion 150.

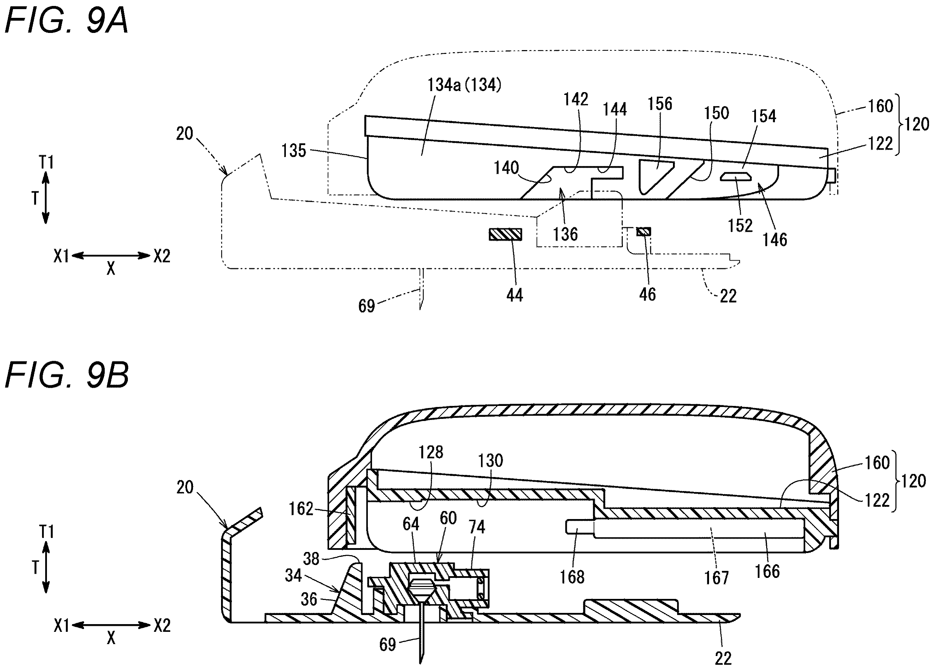

As illustrated in Fig. 8B, at the time point when the guide rib 44 reaches the first insertion and removal opening 138, the top portion 64 of the housing 62 is not exposed from the insertion recess 128. In this state, when the user moves the device main body 100 in the detachment direction X2, the end wall portion 162 interferes with the top portion 38 of the protection portion 34. Thereafter, the device main body 100 is guided in an inclined direction along the inclination of the end surface 36 by the end surface 36 in the connection direction X1 of the protection portion 34. Consequently, while continuing the oblique movement, the device main body 100 moves further away from the holder 20, as illustrated in Fig. 9A. Through the above operations, interference of the casing 120 with the cannula unit 60 during detachment of the device main body 100 from the holder 20 is prevented. As illustrated in Fig. 9A, the guide rib 44 is detached from the guide groove 136 via the first insertion and removal opening 138, and the protrusion 46 is detached from the recess 146 via the second insertion and removal opening 148.

As described above, in the side view from the width direction W, by sliding the device main body 100 on the holder 20 from a position where the portion of the end side in the detachment direction X2 of the device main body 100 protrudes in the detachment direction X2 from the upper surface of the holder 20, and thereby connecting the drug solution flow path 167, the sliding distance of the device main body 100 is shortened. Thus, by partially sliding the device main body 100 relative to the holder 20 for connection, the stroke required for the sliding operation is reduced. Therefore, the operation of connecting the device main body 100 to the holder 20 is facilitated.

Further, when the device main body 100 is detached from the holder 20, the protection portion 34 can prevent interference of the cannula unit 60 with the casing 120. Therefore, even when the drug solution administration device 10 is mounted to a site that the patient is difficult to visually confirm, such as the back or waist, the device main body 100 can still be reliably and easily detached from the holder 20.

The present embodiment has the following effects.

The drug solution administration device 10 includes the holder 20 that is adhered to the body BD of the patient, the cannula unit 60 that is provided on the holder 20, and the device main body 100 that is detachably mounted to the holder 20. The device main body 100 includes the reservoir 102 and the casing 120 accommodating the reservoir 102. The reservoir 102 is filled with the drug solution to be supplied to the cannula 66 of the cannula unit 60.

The holder 20 includes the base portion 22. The base portion 22 includes the holder outer surface 24 that faces the body BD when the holder 20 is adhered to the body BD, and the holder inner surface 26 that is opposite to the holder outer surface 24. The holder inner surface 26 faces the device main body 100 when the device main body 100 is mounted to the holder 20.

The cannula unit 60 includes the housing 62 that is mounted to the holder inner surface 26 in a state of protruding from the holder outer surface 24 in the thickness direction T of the base portion 22, and the first connecting portion 74 that is provided on the housing 62.

The device main body 100 includes the second connecting portion 168 that faces the first connecting portion 74. By the device main body 100 moving relative to the holder 20 in the connection direction X1 along the holder inner surface 26, the first connecting portion 74 and the second connecting portion 168 are connected to each other. In contrast, by the device main body 100 moving relative to the holder 20 in the detachment direction X2 along the holder inner surface 26, the first connecting portion 74 and the second connecting portion 168 are detached from each other. Here, the detachment direction X2 is the direction opposite to the connection direction X1. The second connecting portion 168 may be a needle tube.

One of the holder 20 and the casing 120 includes the guide rib 44. The other of the holder 20 and the casing 120 includes the guide groove 136 with which the guide rib 44 is engaged in a relatively movable manner. At least one of the guide rib 44 and the guide groove 136 includes the first inclined portion 140 that is inclined in a direction away from the base portion 22 as the first inclined portion 140 extends in the detachment direction X2. In the above embodiment, a mode is illustrated in which the holder 20 includes the guide rib 44, the casing 120 includes the guide groove 136, and the guide groove 136 includes the first inclined portion 140.

In the above configuration, when the device main body 100 is detached from the holder 20, the guide rib 44 and the first inclined portion 140 guide the device main body 100 in an inclined direction along the first inclined portion 140.

When the device main body 100 is removed from the holder 20, the guide rib 44 moves relatively along the guide groove 136. During this movement, the guide rib 44 and the first inclined portion 140 guide the device main body 100 in a direction inclined relative to the detachment direction X2. That is, during attachment and detachment of the device main body 100 with respect to the holder 20, the moving direction of the device main body 100 is regulated. As a result, interference of the casing 120 with the cannula unit 60 is prevented. Accordingly, detachment of the cannula unit 60 from the holder 20 is prevented. In addition, separation of the holder 20 from the body BD is prevented, and removal of the cannula 66 from the body BD is prevented.

The holder 20 includes the two holder side portions 40 (the first side portion 40a and the second side portion 40b) that protrude from the base portion 22 in the thickness direction T. The two holder side portions 40 are provided at positions sandwiching the casing 120 when the device main body 100 is mounted to the holder 20. Each of the two holder side portions 40 includes the guide rib 44 or the guide groove 136.

In this case, the holder 20 includes either two guide ribs 44 or two guide grooves 136, and the casing 120 includes the other of the two guide ribs 44 and the two guide grooves 136. By guiding the two guide ribs 44 with two first inclined portions 140, the moving direction of the device main body 100 can be regulated more effectively. In the above embodiment, a mode is illustrated in which each of the two holder side portions 40 includes the guide rib 44.

The holder 20 (or the casing 120) includes the engagement recess 144 that is continuous with the end portion of the guide groove 136 facing the detachment direction X2, and extends in the detachment direction X2. When the first connecting portion 74 and the second connecting portion 168 are connected to each other, the guide rib 44 engages with the engagement recess 144.

By the guide rib 44 engaging with the engagement recess 144, detachment of the device main body 100 from the holder 20 is prevented. That is, it is easy to maintain the state in which the device main body 100 is mounted to the holder 20. Furthermore, by relatively moving the guide rib 44 along the engagement recess 144, the first connecting portion 74 and the second connecting portion 168 can be easily connected and detached.

The holder 20 (or the casing 120) includes the protrusion 46. The casing 120 (or the holder 20) includes the recess 146. The protrusion 46 is inserted into the recess 146 in a relatively movable manner in an inclined direction.

According to this configuration, when the device main body 100 is attached to or detached from the holder 20, the protrusion 46 can be guided along the inner surface of the recess 146. In this case, the moving direction of the device main body 100 relative to the holder 20 is regulated more effectively. As a result, interference of the cannula unit 60 with the casing 120 is further prevented. In the above embodiment, the protrusion 46 is a locking protrusion for locking the device main body 100 in a state of being mounted to the holder 20. Thus, the protrusion 46 is utilized, for example, for locking and guiding purposes.

The holder 20 includes the protection portion 34 that protrudes in the thickness direction T from the holder inner surface 26. The casing 120 includes the insertion recess 128 into which the protection portion 34 is inserted in a detachable manner. In the state where the device main body 100 is mounted to the holder 20, the protection portion 34 is positioned between the end wall portion 162 that constitutes the end portion of the insertion recess 128 in the connection direction X1 and the housing 62 of the cannula unit 60. When the protruding direction of the protection portion 34 from the holder inner surface 26 is defined as the height direction T1, the height of the top portion 38 of the protection portion 34 is equal to or greater than the height of the top portion 64 of the housing 62.

When the user moves the device main body 100 in the detachment direction X2, the protection portion 34 comes into contact with the end wall portion 162 of the insertion recess 128. Consequently, the device main body 100 stops, and thus, further movement of the device main body 100 in the detachment direction X2 is prevented. Therefore, interference of the casing 120 with the cannula unit 60 is prevented.

In the protection portion 34, the end surface 36 facing the connection direction X1 is an inclined surface that inclines in the detachment direction X2 from the holder inner surface 26 toward the top portion 38.

According to this configuration, after the protection portion 34 comes into contact with the end wall portion 162 of the insertion recess 128, the end surface 36 (inclined surface) guides the device main body 100. The device main body 100 moves in the inclined direction along the end surface 36. Therefore, it is possible to separate the device main body 100 from the holder 20 while preventing interference of the end wall portion 162 of the casing 120 with the cannula unit 60.

The cannula unit 60 is positioned, in the base portion 22, between the two end portions in the axial direction X, which extends in the connection direction X1 and the detachment direction X2, and between the two end portions in the width direction W orthogonal to the axial direction X.

Even when the cannula unit 60 is arranged at the above-described position, interference of the casing 120 with the cannula unit 60 can be prevented when attaching or detaching the device main body 100 to or from the holder 20. That is, the degree of freedom in the layout of the cannula unit 60 is improved.

Note that, the present invention is not limited to the above disclosure, and various configurations can be adopted without departing from the gist of the present invention.

## Claims

1. A drug solution administration device comprising: a holder that is adhered to a body of a living subject; a cannula unit that is provided on the holder; and a device main body that is detachably mounted to the holder, wherein
the device main body includes a reservoir which is filled with a drug solution to be supplied to a cannula of the cannula unit; and a casing which accommodates the reservoir,
the holder includes a base portion,
the base portion includes a holder outer surface which faces the body when the holder is adhered to the body; and a holder inner surface that is on a side opposite to the holder outer surface and faces the device main body when the device main body is mounted to the holder,
the cannula unit includes a housing which is attached to the holder inner surface in a state of protruding in a thickness direction of the base portion from the holder outer surface; and a first connecting portion provided on the housing,
the device main body includes a second connecting portion facing the first connecting portion, the first connecting portion and the second connecting portion are connected to each other by relative movement of the device main body with respect to the holder along the holder inner surface in a connection direction, the first connecting portion and the second connecting portion are detached from each other by relative movement of the device main body with respect to the holder along the holder inner surface in a detachment direction, which is opposite to the connection direction,
one of the holder and the casing includes a guide rib, another of the holder and the casing includes a guide groove with which the guide rib engages in a relatively movable manner,
at least one of the guide rib and the guide groove includes an inclined portion inclined in a direction away from the base portion as the inclined portion extends in the detachment direction, and
when the device main body is attached to or detached from the holder, the guide rib and the inclined portion guide the device main body in an inclined direction along the inclined portion.

2. The drug solution administration device according to claim 1, wherein the holder includes two holder side portions which protrude from the base portion in the thickness direction and are provided at positions sandwiching the casing when the device main body is mounted to the holder,
each of the two holder side portions includes the guide rib or the guide groove.

3. The drug solution administration device according to claim 1, wherein the holder or the casing includes an engagement recess which is continuous with an end portion of the guide groove facing the detachment direction and extends in the detachment direction,
and when the first connecting portion and the second connecting portion are connected to each other, the guide rib engages with the engagement recess.

4. The drug solution administration device according to claim 1, wherein one of the holder and the casing includes a protrusion, and another of the holder and the casing includes a recess into which the protrusion is inserted in a relatively movable manner in the inclined direction.

5. The drug solution administration device according to claim 1, wherein the device main body is detached from the holder by sliding along the holder inner surface in the detachment direction so that at least a part of the device main body protrudes from an end portion of the holder in the detachment direction, and then moving in a direction inclined with respect to the detachment direction.

6. The drug solution administration device according to claim 1, wherein the holder includes a protection portion which protrudes in the thickness direction from the holder inner surface,
the casing includes an insertion recess into which the protection portion is inserted in a detachable manner,
in a state where the device main body is mounted to the holder, the protection portion is positioned between an end wall portion constituting an end portion of the insertion recess in the connection direction and the housing of the cannula unit, and
when a protruding direction of the protection portion from the holder inner surface is defined as a height direction, a height of a top portion of the protection portion is equal to or greater than a height of a top portion of the housing.

7. The drug solution administration device according to claim 6, wherein an end surface of the protection portion facing the connection direction (is an inclined surface inclined in the detachment direction from the holder inner surface toward the top portion of the protection portion.

8. The drug solution administration device according to any one of claims 1 to 7, wherein the cannula unit is positioned, in the base portion, between two end portions in an axial direction which extends in the connection direction and the detachment direction, and between two end portions in a width direction orthogonal to the axial direction.
